# EUROPÄISCHE PATENTSCHRIFT

(11) **EP 0 677 278 B1**
(45) Veröffentlichungstag und Bekanntmachung des Hinweises auf die Patenterteilung: **22.05.2002**
(21) Anmeldenummer: 95104528.5
(22) Anmeldetag: 28.03.1995
(51) Int. Cl.: A61B 19/00

(54) **Stereotaktischer Adapter sowie Verfahren zu dessen Betrieb**
Stereotactic adapter and method for its use
Adapteur stéréotactique et procédé pour son opération

(30) Priorität: 13.04.1994 DE 4412605
(43) Veröffentlichungstag der Anmeldung: 18.10.1995
(73) Patentinhaber: Carl Zeiss, 89518 Heidenheim (Brenz) (DE)
(72) Erfinder: Luber, Joachim, D-73457 Essingen-Forst (DE); Mackevics, Arvids, D-73432 Aalen-Unterkochen (DE); Mick, Franz, D-37133 Friedland (DE); Bauer, Bernhard Ludwig, Prof. Dr., D-35033 Marburg (Lahn) (DE)

(56) Entgegenhaltungen:
- WO-A-83/03343
- WO-A-92/20295
- DE-A- 4 134 481
- DE-A- 4 202 922
- DE-U- 9 204 118
- US-A- 3 910 276
- US-A- 5 078 140
- US-A- 5 209 219
- US-A- 5 295 477

## Beschreibung

Die vorliegende Erfindung betrifft eine Vorrichtung zur rechnergestützten Chirurgie umfassend einen stereotaktischen Adapter zur Anordnung eines chirurgischen Therapie- und/oder Diagnoseinstrumentes an einem Operationsmikroskop, welches wiederum an einem motorischen Trägersystem befestigt ist und damit positioniert wird. Gegenstand der vorliegenden Erfindung ist desweitere einen stereotaktischen Adapter.

In der rechnergestützen stereotaktischen Chirurgie sind verschiedenste Vorrichtungen zum definierten Positionieren von chirurgischen Therapie- und/oder Diagnoseinstrumenten bekannt. Stellvertretend sei hierbei etwa auf die US 5,228,429, US 5,078,140, US 5,142,930, US 5,050,608 sowie die DE 42 02 922 der Anmelderin verwiesen.

In den dort beschriebenen Vorrichtungen werden verschiedenste chirurgische Therapie- und/oder Diagnoseinstrumente wie Endoskope, Biopsienadeln, Operationsmikroskope etc. an einem mehrgelenkigen Trägersystem angeordnet, das entsprechende Winkelencoder für die einzelnen Gelenke umfaßt, die über eine zentrale Steuereinheit ausgewertet werden. Mitunter weisen die Trägersysteme auch Antriebe für die verschiedenen Gelenk-Achsen auf. Mit Hilfe der Trägersysteme ist nunmehr ein definiertes Positionieren des daran angeordneten Instrumentes im jeweiligen Geräte-Koordinatensystem in mehreren räumlichen Freiheitsgraden möglich.

Soll mit Hilfe dieser Vorrichtungen ein stereotaktischer Eingriff an einem Patienten mit einem daran angeordneten chirurgischen Therapie- und/oder Diagnoseinstrument vorgenommen werden, so ist zunächst ein Referenzieren zwischen dem Patienten-Koordinatensystem und dem Geräte-Koordinatensystem erforderlich, um anschließend ein definiertes Positionieren des jeweils verwendeten Instrumentes zu gewährleisten. In der US 5,228,429 erfolgt dies beispielsweise dergestalt, daß die Spitze des zu positionierenden Endoskopes jeweils in Kontakt mit den zu behandelnden Körperteilen des Patienten gebracht und über mehrere derartiger Antastungen schließlich auf die Position oder die Größe dieses Körperteiles rückgeschlossen wird. Offensichtlich ist dieses Verfahren zum Referenzieren der unterschiedlichen Koordinatensysteme als Voraussetzung für den stereotaktischen Einsatz des Endokospes mit Gefahren für den Patienten verbunden, insbesondere wenn etwa in der Gehirn-Region des Patienten referenziert werden soll.

Neben der Referenzierung von Patienten- und Gerätekoordinatensystem ist ggf. noch ein Referenzieren mit präoperativ gewonnenen Diagnosebildern, d.h. dem entsprechenden Bildkoordinatensystem zusätzlich erforderlich, wenn diese bildgebenden Informationen ebenfalls zur Gerätesteuerung herangezogen werden sollen.

Aufgabe der vorliegenden Erfindung ist es, eine Vorrichtung sowie ein Verfahren zu schaffen, daß die oben genannten Nachteile vermeidet und einen gefahrlosen stereotaktischen Betrieb verschiedenster Therapie- und/oder Diagnoseinstrumente ermöglicht, die über ein motorisches Trägersystem positionierbar sind.

Diese Aufgabe wird gelöst durch eine Vorrichtung mit den Merkmalen des Anspruches 1 sowie einen Adapter gemäß Anspruch 12.

Erfindungsgemäß ist ein stereotaktischer Adapter an einem Operationsmikroskop vorgesehen, an dem das jeweilige chirurgische Therapie- und/oder Diagnoseinstrument in einer definierten Relativposition angeordnet wird. Das Operationsmikroskop seinerseits wiederum ist an einem motorischen Trägersystem angeordnet, über das eine Positionierung des Operationsmikroskopes in mehreren räumlichen Freiheitsgraden möglich ist. Der erfindungsgemäße Adapter gewährleistet dabei die reproduzierbare, geometriemäßig bekannte Anordnung des verwendeten chirurgischen Therapieund/oder Diagnoseinstrumentes am Operationsmikroskop. Aufgrund der bekannten geometrischen Abmessungen des Operationsmikroskopes, des verwendeten Adapters sowie des eingesetzten Instrumentes ist mit Hilfe des erfindungsgemäßen stereotaktischen Adapters nunmehr ein gefahrloses, definiertes Positionieren des verwendeten chirurgischen Therapie- und/oder Diagnoseinstrumentes mit Hilfe des motorischen Trägersystemes möglich. Die Koordinaten des Einsatzortes des jeweiligen Instrumentes werden aus den jeweiligen bekannten Geometrie-Informationen sowie den gelieferten Encoderinformationen des motorischen Trägersystemes über die zentrale Steuereinheit laufend aktuell bestimmt.

Neben der nunmehr für den Patienten gefahrlosen Referenzierung der unterschiedlichen Koordinatensysteme und damit auch des verwendeten Instrumentes resultiert als weiterer Vorteil beim Einsatz des erfindungsgemäßen stereotaktischen Adapters die Möglichkeit für den Chirurgen, interessierende Details im Operationsfeld über das Operationsmikroskop zu beobachten. Z.B. kann etwa ein kleiner Schnitt, durch den etwa ein Endoskop eingeführt werden soll, rasch visuell inspiziert werden.

Das Referenzieren des jeweiligen chirurgischen Therapie- und/oder Diagnoseinstrumentes erfolgt z.B. durch entsprechendes Positionieren bzw. optisches Referenzieren des Operationsmikroskopes wie es in der DE 41 34 481 der Anmelderin beschrieben ist und auf die hiermit ausdrücklich Bezug genommen wird. In vorteilhafter Weise werden dabei die Navigationsmöglichkeiten des Operationsmikroskopes mit seinen Detektoren und dem Positionserkennungssystem auf optischer Basis ausgenutzt. Aufgrund der bekannten geometrischen Abmessungen des Operationsmikroskopes, des Instrumentes sowie des Adapters wird damit gleichzeitig auch das Referenzieren des jeweiligen Instrumentes bewerkstelligt.

Neben einem derartigen Positionserkennungssytem auf optischer Basis können auch geeignete akustische Positionserkennungssyteme eingesetzt werden, mit denen sowohl die Patientenkoordinaten als auch die Operationsmikroskop-Koordinaten dreidimensional mit hoher Präzision bestimmt werden können.

Weitere Vorteile sowie Einzelheiten des erfindungsgemäßen stereotaktischen Adapters und eines Verfahrens zu seinem Betrieb ergeben sich aus der nachfolgenden Beschreibung von Ausführungsbeispielen anhand der beiliegenden Figuren.

Dabei zeigt
- Fig. 1: eine Prinzip-Darstellung eines Ausführungsbeispieles des erfindungsgemäßen stereotaktischen Adapters, angeordnet an einem geeigneten Operationsmikroskop, welches wiederum über ein motorisches Trägersystem positioniert wird;
- Fig. 2: das Operationsmikroskop inclusive des erfindungsgemäßen stereotaktischen Adapters aus Fig. 1 in einer Seitenansicht;
- Fig. 3: eine Schnitt-Darstellung des stereotaktischen Adapters aus Fig. 2;
- Fig. 4: ein Blockschaltbild zum Verfahrensablauf eines Ausführungsbeispieles eines Verfahrens, das mit Hilfe des stereotaktischen Adapters aus den Fig. 1-3 durchgeführt wird.

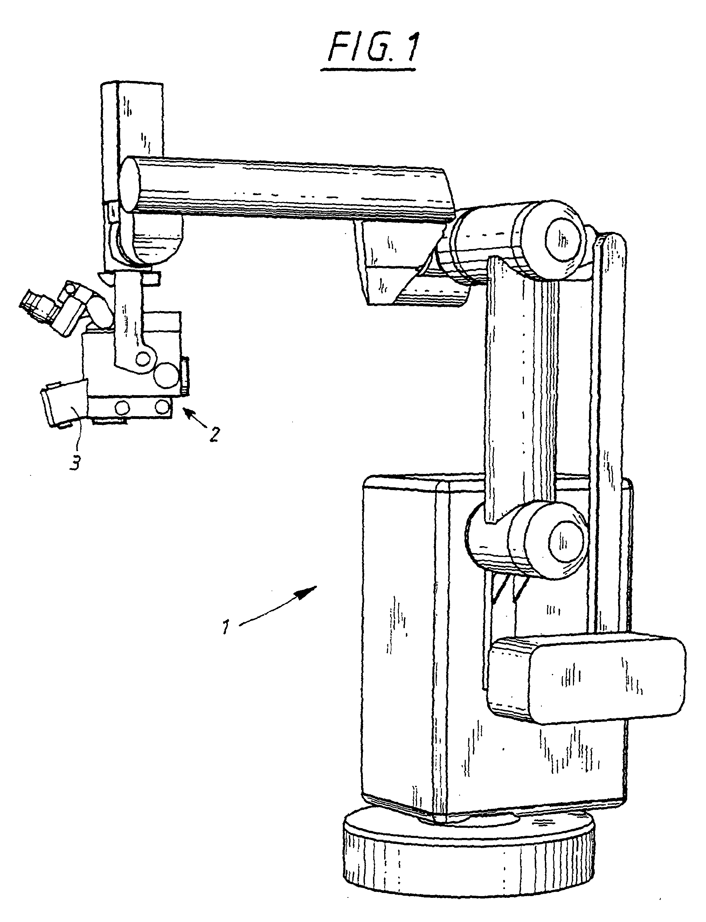

in Fig. 1 ist ein mögliches Ausführungsbeispiel des erfindungsgemäßen stereotaktischen Adapters (3) dargestellt, der an einem geeigneten Operationsmikroskop (2) angeordnet ist. Das Operationsmikroskop (2) seinerseits ist wiederum an einem motorischen Trägersystem (1) angeordnet, wie es z.B. aus der DE 42 02 922 der Anmelderin bekannt ist.

Das motorische Trägersystem (1) weist eine Reihe von gelenkig miteinander verbundenen Elementen auf, wobei die den einzelnen Achsen zugeordneten Gelenke motorisch angetrieben werden und jeweils ein oder mehrere Winkelencoder umfassen. Mit dem motorischen Trägersystem (1) ist eine definierte motorische Positionierung der daran angeordneten Instrumente in bis zu sechs räumlichen Freiheitsgraden mit hoher Positioniergenauigkeit möglich.

Die jedem Gelenk des motorischen Trägersytemes (1) zugeordneten Winkelencoder liefern Positions-Informationen hinsichtlich der aktuellen Stellung der jeweiligen Gelenke an eine zentrale Steuereinheit, die im dargestellten Ausführungsbeispiel im Sockelteil des motorischen Trägersystemes (1) angeordnet und demzufolge in Fig. 1 nicht sichtbar ist. Auf Grundlage der bekannten Geometrie-Informationen des motorischen Trägersystemes (1) sowie der Encoder-Informationen kann damit stets die exakte, aktuelle räumliche Position des daran angeordneten Operationsmikroskopes bzw. die aktuelle Sehfeldposition bestimmt werden.

Hinsichtlich einer detaillierten Beschreibung des motorischen Trägersystemes (1) sei an dieser Stelle ausdrücklich auf die bereits oben erwähnte DE 42 02 922 der Anmelderin verwiesen.

Ein Operationsmikroskop (2), das sich in vorteilhafer Weise für eine derartige Anordnung bzw. einen derartigen Einsatz eignet, ist aus der DE 41 34 481 der Anmelderin bekannt. Dieses Operationsmikroskop (2) weist Detektoren zum Erfassen der optischen Systemdaten wie Vergrößerung und Fokussierstellung sowie ein integriertes Positionserkennungssystem auf optischer Basis auf, so daß über die zentrale Steuereinheit stets sowohl die exakte räumliche Position und Orientierung des Operationsmikroskopes (2) als auch des jeweils betrachteten Sehfeldes bekannt ist.

Wie bereits oben angedeutet, kann anstelle des integrierten optischen Positionserkennungssytemes beispielsweise auch ein akustisches Positionserkennungssytem auf Ultraschall-Basis erfindungsgemäß eingesetzt werden. Derartige Systeme sind etwa als sogenante 3D-Digitizer bekannt.
Das Operationsmikroskop (2) umfaßt ferner eine Einspiegelungsvorrichtung, über welche die Einspiegelung diversester Bilder aus präoperativen Diagnose-Verfahren oder anderweitig geeignet aufbereiteter Bilder möglich ist. Dem Chirurgen wird durch die überlagerte Darstellung des jeweils eingesehenen Sehfeldes mit diesen Bildern damit eine Orientierungshilfe während der Operation angeboten.

Am Operationsmikroskop (2) ist erfindungsgemäß der stereotaktische Adapter (3) angeordnet, der wiederum eine eindeutig reproduzierbare Anordnung eines chirurgischen Therapie- und/oder Diagnoseinstrumentes am Operationsmikroskop (2) und damit am motorischen Trägersystem (1) ermöglicht. In der Darstellung von Fig. 1 ist im erfindungsgemäßen Adapter (3) dabei kein chirurgisches Therapie- und/oder Diagnoseinstrument vorgesehen.

Der erfindungsgemäße stereotaktische Adapter (3) gestattet nunmehr den rechnergestützten Einsatz eines Operationsmikroskopes (2) in Verbindung mit einem chirurgischen Therapieund/oder Diagnoseinstrument, wobei über das motorische Trägersystem eine definierte räumliche Positionierbarkeit in mindestens drei räumlichen Freiheitsgraden möglich sein sollte. Das dargestellte, aus der DE 42 02 922 bekannte Ausführungsbeispiel des motorischen Trägersystemes (1) ermöglicht sogar die Positionierung in insgesamt sechs räumlichen Freiheitsgraden, d.h. drei Translations- und drei Rotationsfreiheitsgraden.

Durch Ausnutzen der optischen Referenzierungs-Verfahren des Operationsmikroskopes kann numehr gleichzeitig eine kontaktlose Referenzierung des verwendeten Instrumentes und damit ein gefahrloser stereotaktischer Einsatz desselben realisiert werden.

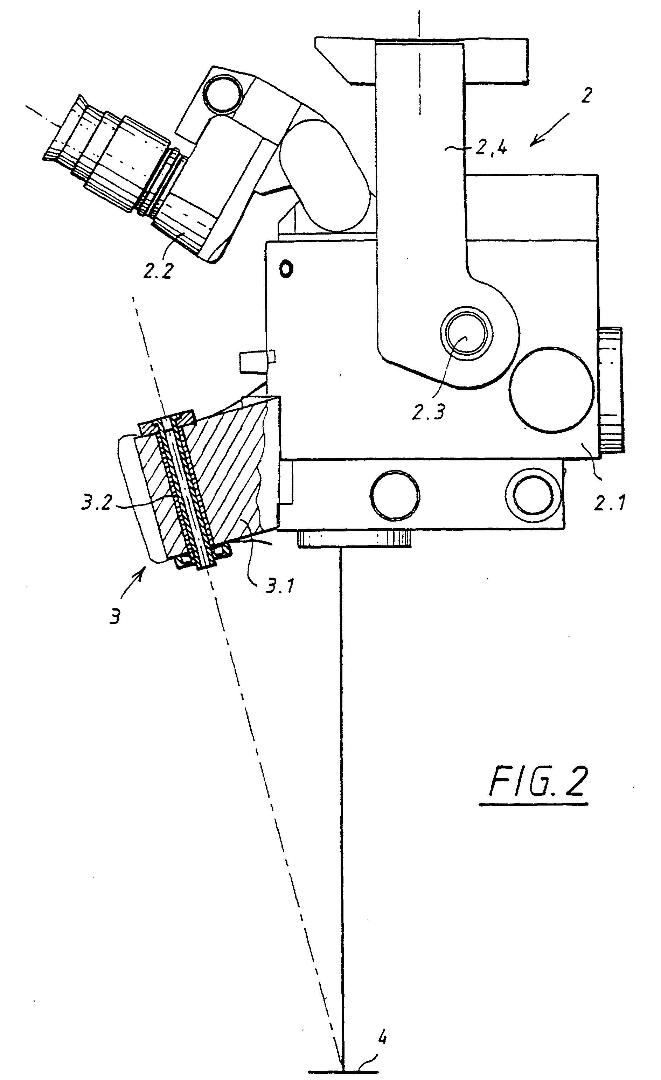

Der Aufbau des erfindungsgemäßen stereotaktischen Adapters wird anhand der Fig. 2 näher erläutert, wo eine Seitenansicht des Operationsmikroskopes (2) aus Fig. 1 mit einer Teil-Schnittdarstellung des erfindungsgemäßen Adapters (3) dargestellt ist.
Das über ein Verbindungselement (2.4) am motorischen Trägersystem angeordnete Operationsmikroskop (2) umfaßt ein Mikroskopkörper-Gehäuse (2.1), in dem die - nicht dargestellte - komplette Optik inclusive Detektoren und
Positionerkennungssystem angeordnet sind. Zum detaillierten Aufbau des vorzugsweise zu verwendenden Operationsmikroskopes (2) sei auf die bereits zitierte DE 41 34 481 der Anmelderin verwiesen.

Oberhalb des Mikroskopkörper-Gehäuses (2.1) ist ein an sich bekannter Binokulartubus (2.2) angeordnet, der relativ zum Mikroskopkörper-Gehäuse (2.1) des Operationsmikroskopes (2) schwenkbar ist. Das Operationsmikrokop (2) weist desweiteren eine gelenkige Schnittstelle (2.3) auf, an der es über ein Verbindungselement (2.4) am motorischen Trägersystem befestigt wird.
Am unteren Teil des Mikroskopkörper-Gehäuses (2.1) ist der erfindungsgemäße stereotaktische Adapter (3) am Operationsmikroskop (2) befestigt. Erforderlich ist hierbei eine starre Verbindung zwischen dem Adapter (3) und dem Operationsmikroskop (2), da nur somit eine definierte Relativanordnung zueinander gewährleistet ist.

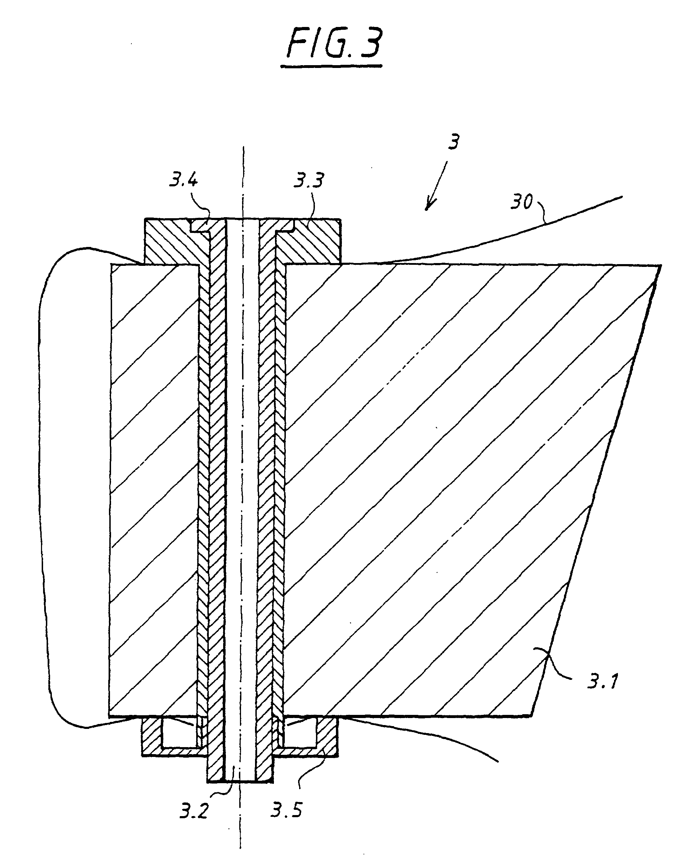

Die Verbindung zwischen dem Operationsmikroskop (2) und dem Adapter (3) kann sowohl fest als auch lösbar gestaltet werden, wesentlich ist jeweils nur eine starre Verbindung. Möglich ist dabei etwa eine Schraubverbindung etc. .
Der stereotaktische Adapter (3) am Operationsmikroskop (2) besteht aus einem Grundkörper (3.1) mit einer zylinderförmigen Bohrung (3.2), in die das jeweilige chirurgische Therapieund/oder Diagnoseinstrument einführbar ist. Zum weiteren Aufbau des erfindungsgemäßen Adapters sei auf die nachfolgende Beschreibung der Fig. 3 verwiesen.

Der stereotaktische Adapter (3) ist so am Operationsmikroskop (2) angeordnet, daß die zylinderförmige Bohrung (3.2) und damit auch das darin einzuführende chirurgische Therapie- und/oder Diagnoseinstrument in eine definierte, bekannte Richtung ausgerichtet ist.
Die betrachtete Sehfeldebene (4) und das chirurgische Therapieund/oder Diagnose-Instrument bzw. dessen Zielort werden demnach durch den Adapter (3) reproduzierbar relativ zueinander ausgerichtet. Ist die exakte Lage der Sehfeldebene (4) in der Art und Weise einmal bestimmt, wie es in der DE 41 34 481 beschrieben wird, so ist damit der zentralen Steuereinheit auch der Zielort des jeweils verwendeten Instrumentes koordinatenmäßig bekannt.

Zur Zielortbestimmung muß lediglich noch erfaßt werden, wie weit das jeweilige Instrument in die zylinderförmige Bohrung eingeführt wird und welche Abmessungen das jeweilige Instrument aufweist.
Wird etwa ein Endoskop als chirurgisches Therapie- und/oder Diagnoseinstrument im stereotaktischen Adapter eingesetzt, so wird unter dessen Zielort dabei der Punkt verstanden, wo die Einführung in den Patienten erfolgt.

Die zur exakten Zielort-Bestimmung desweiteren noch erforderlichen Informationen zur Geometrie des jeweiligen Instrumentes, das in den stereotaktischen Adapter eingesetzt wird, können der zentralen Steuereinheit nunmehr auf verschiedene Art und Weise zugänglich gemacht werden.
So ist es etwa einerseits möglich, daß diese Instrumenten-Geometrie-Informationen vom Benutzer über eine Eingabeschnittstelle manuell eingegeben werden.
Alternativ können diese Informationen der Steuereinheit jedoch auch automatisiert übergeben werden. Hierzu weist der Adapter ein Kodierungs-Erkennungssystem auf, anhand dessen eine eindeutige Identifikation des jeweiligen Instrumentes automatisiert erfolgt. Diese Identifikations-Informationen werden anschließend der zentralen Steuereinheit übergeben, wo Geometrie-Informationen zu einer Reihe verschiedenster Instrumente bereitgehalten werden, die dann anhand der übergebenen Identifikations-Informationen zugeordnet werden können.

Als geeignete Kodierungs-Erkennungsysteme kommen dabei z.B. bekannte optische Kodierungssysteme wie etwa sog. Bar-Code-Systeme oder dgl. in Frage. Möglich ist hierbei jedoch auch der Einsatz magnetischer Kodierungssysteme etc..

In Fig. 3 ist eine nochmals vergrößerte seitliche Schnitt-Ansicht des erfindungsgemäßen Adapters (3) dargestellt. Im Grundkörper (3.1) des Adapters (3) ist eine zylinderförmige Bohrung (3.2) vorgesehen, in die das eigentliche Instrument, z.B. ein Endoskop, eingeführt wird. Für die zylinderförmige Bohrung im Adapter werden nunmehr eine Reihe von zylinderförmigen Hülsen mit unterschiedlichen Zylinderdurchmessern, Hülsenlängen etc. bereitgehalten. In Fig. 3 sind zwei dieser Hülsen (3.3, 3.4) in der zylinderförmigen Bohrung (3.2) des Adapters (3) angeordnet. Die Hülsen (3.3, 3.4) unterschiedlichen Durchmessers dienen als Anpass-Elemente, um verschiedenste Instrumente in einem einzigen Adapter einsetzen zu können. Unterschiedliche Istrumente wie etwa Endoskope und Biopsienadeln oder aber auch verschiedene Endoskope mit variierenden Optik-Durchmessern können mit Hilfe dieser Anpass-Elemente in vorteilhafter Weise im gleichen Adapter eingesetzt werden ohne jeweils einen separaten Adapter zu erfordern. Es müssen vor dem jeweiligen Einsatz lediglich die passenden Hülsen eingewechselt werden.
Im dargestellten Ausführungsbeispiel der Fig. 3 ist ferner an der Unterseite des Adapters ein Sicherungselement (3.5) vorgesehen, das zur sicheren Befestigung der eingesetzten Hülsen (3.3, 3.4) dient.

Weitere Sicherungselemente sind erforderlich, um auch das jeweils in den Adapter eingesetzte Instrument gegen ein Herausfallen zu sichern und die definierte Relativanordnung des Instrumentes zum Adapter bzw. dem Operationsmikroskop zu gewährleisten. Hierfür kann das Instrument z.B. mit dem Adapter verschraubt werden.
Weiterhin ist in Fig. 3 ein Teil eines Drapes (30) dargestellt, mit dem sowohl das Operationsmikroskop als auch der Adapter (3) während der Operation bedeckt werden. Das Drape (30) weist an den Stellen, wo das jeweilige Instrument in den Adapter hinein-bzw. herausragt entsprechende Öffnungen auf. Ferner muß das Drape (30) gegen Verrutschen gesichert werden, um die richtige Position dieser beiden Öffnungen sicherzustellen. Beispielsweise kann das Drape (30) hierzu am Adapter über die Hülsen (3.3, 3.4) und/oder die Sicherungselemente (3.5) geeignet eingeklemmt werden.
Aus Sterilitätsgründen ist es ferner vorteilhaft, die verwendeten Hülsen zur Anpassung an verschiedenste Instrument-Geometrien sterilisierbar zu wählen. Z.B. können die Hülsen mit einem sterilen Überzug versehen werden oder aber materialmäßig so gefertigt werden, daß eine einfache Sterilisationsmöglichkeit über einen Autoklaviatoren möglich ist.

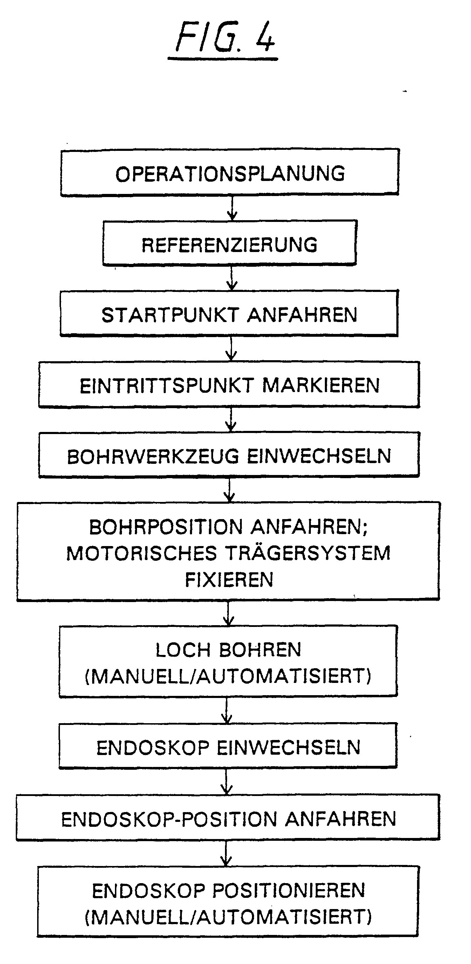

Anhand des in Fig. 4 dargestellten Flußdiagrammes erfolgt die Beschreibung eines möglichen Verfahrens zum Betrieb des erfindungsgemäßen Adapters in Verbindung mit einem Bohrer und einem Endoskop bei einer Gehirnoperation.
Vor der eigentlichen Operation erfolgt dabei die Operationsplanung durch den Chirurgen an der zentralen Steuereinheit. Anhand der präoperativ generierten Diagnosedaten, z.B. CT-Bilder etc., wird dabei der Eintrittspunkt des Endoskopes im Patienten definiert festgelegt.

Anchließend wird mit Hilfe des Operationsmikroskopes aus der DE 41 34 481 eine optische Referenzierung zwischen dem Patienten-, dem Geräte- und dem Bild-Koordinatensystem durchgeführt. Hierzu werden beispielsweise drei sogenannte Marker am Patienten über nacheinander erfolgende Eichmessungen mit Hilfe des optischen Positions-Erkennungssystemes vermessen. Die zentrale Steuereinheit übernimmt anhand der Informationen aus dieser Eichmessung die Korrelation der verschiedenen Koordinatensysteme: Geräte-Koordinatensystem, Bild-Koordinatensystem und Patienten-Koordinatensystem.
Anschließend verfährt das motorische Trägersystem das Operationsmikroskop an den Startpunkt gemäß der erfolgten Operationsplanung, so daß der Endoskop-Eintrittspunkt in der Sehfeldmitte (4) des Operationsmikrsokopes liegt. An dieser Stelle ist es z.B. möglich, durch Einspiegelung der vorher bearbeiteten Diagnosebilder das erforderliche Bohrloch am Patienten dem Sehfeld zu überlagern. Im nächsten Verfahrensschritt überträgt der Chirurg nunmehr die Position bzw. Form des erforderlichen Bohrloches, auf die Haut des Patienten, indem diese etwa manuell oder aber mittels eines Lasers auf der Haut des Patienten markiert wird.

Nach der Markierung des Eintrittspunktes wird das erfoderliche Bohrwerkzeug in den Adapter eingewechselt, wobei aus Sicherheitsgründen das Operationsmikroskop hierzu in eine größere Entfernung vom Patienten weg verfahren wird.
Daraufhin wird die Bohrposition angefahren und das motorische Trägersystem in dieser Position fixiert.
Anschließend ist es möglich, das erforderliche Bohrloch entweder manuell - geführt durch den Adapter - oder automatisiert an der markierten Stelle zu setzen. Im letzteren Falle erfolgt eine neue Ausrichtung des motorischen Trägersystems bzw. des daran angeordneten Operationsmikroskopes inklusive Adapter und Bohrer und das anschließende stereotaktische Bohren des erforderlichen Bohrloches.
Nach diesem Verfahrensschritt, d.h. dem Setzen des erforderlichen Bohrloches auf stereotaktisch-automatisierte oder aber manuelle Art und Weise wird der Bohrer aus dem Adapter entfernt und das Endoskop oder gegebenenfalls ein anderes medizinisches Therapie- oder Diagnoseinstrument in den Adapter eingeführt und eingewechselt.

Anschließend wird über das motorische Trägerystem die erforderliche Endoskop-Position angefahren. Das nunmehr folgende Einführen bzw. Positionieren des Endoskopes oder ggf. anderweitig vorgesehenen Instrumentes kann dann manuell oder aber wieder automatisiert über das motorische Trägersystem erfolgen.
Beim manuellen Einführen des Instrumentes in den Patienten verfährt das motorische Trägersystem vor diesem Schritt das Operationsmikroskop bzw. den Adapter in die erforderliche Entfernung respektive Position, die notwendig ist, damit beim manuellen Einführen des Instrumentes bis zu einem mechanischen Anschlag im Adapter der gewünschte Zielpunkt erreicht ist.
Nachdem das motorische Trägersystem diese Position eingenommen hat, behält es diese Position durch Feststellen der jeweiligen Bremsen, bis der Chirurg das Instrument entsprechend manuell eingeführt hat.

Zum Anfahren dieser Position benötigt die Steuereinheit die bei der Operationsplanung vorgegebenen Koordinaten-Informationen ebenso wie die Geometrie-Informationen zum Adapter bzw.
Instrument, die entweder manuell oder aber automatisiert der zentralen Steuereinheit übergeben wurden.

Alternativ zum manuellen stereotaktischen Einführen des jeweiligen Instrumentes ist es aber auch möglich, das Endoskop über das motorische Trägersystem geführt automatisiert in das gesetzte Bohrloch im Patienten einzuführen. Hierzu wird das Endoskop fest bzw. definiert mit dem Operationsmikroskop bzw. dem Adapter verbunden und entsprechend arretiert. Anschließend verfährt das motorische Trägersystem das Endoskop definiert entlang des vorab geplanten Verfahrweges, die Position des Instrumentes bzw. dessen Spitze ist der zentralen Steuereinheit stets bekannt.

Beim automatisierten Einführen des Instrumentes auf diese Art und Weise ist es vorteilhaft, eine Sicherungseinrichtung vorzusehen, die etwa bei Hindernissen auf dem Verfahrweg automatisch das Verfahren des Instrumentes stoppt und erst nach erfolgter Bestätigung durch den Chirurgen eine weitere Positionierung des Instrumentes ermöglicht. Hierfür können etwa bekannte Doppler-Systeme eingesetzt werden.

## Patentansprüche

1. Vorrichtung zur rechnergestützten Chirurgie, umfassend
ein Operationsmikroskop (2) mit einem Positionserkennungssystem,
ein das Operationsmikroskop (2) tragendes Trägersystem (1), das ein Positionieren des Operationsmikroskopes (2) in mindestens drei räumlichen Freiheitsgraden erlaubt, und eine zentrale Steuereinheit,
**gekennzeichnet durch**
einen stereotaktischen Adapter (3) zur Anordnung eines chirurgischen Therapieund/oder Diagnoseinstrumentes an dem Operationsmikroskop (2), wobei der Adapter (3) eine reproduzierbare Anordnung des jeweiligen chirurgischen Therapie- und/oder Diagnoseinstrumentes am beobachtungswirksamen Operationsmikroskop (2) gewährleistet.

2. Vorrichtung nach Anspruch 1, wobei der Adapter (3) und/oder das Operationsmikroskop (2) mit einem Kodierungs-Erkennungssystem ausgestattet sind, das eine Kodierung des jeweiligen chirurgischen Therapie- und/oder Diagnoseinstrumentes identifiziert und der zentralen Steuereinheit diese Informationen übergibt und die Steuereinheit anhand der übergebenen Informationen eine eindeutige Identifizierung des jeweiligen Instrumentes und der jeweiligen Gerätegeometrie vornimmt.

3. Vorrichtung nach Anspruch 2, wobei ein optisches Kodierungssystem vorgesehen ist.

4. Vorrichtung nach Anspruch 2, wobei ein magnetisches Kodierungssystem vorgesehen ist.

5. Vorrichtung nach Anspruch 1, wobei die zentrale Steuereinheit eine Eingabe-Schnittstelle zur manuellen Identifikation des jeweils verwendeten chirurgischen Therapie- und/oder Diagnoseinstrumentes umfaßt.

6. Vorrichtung nach Anspruch 1, wobei als chirurgisches Therapie- und/oder Diagnoseinstrument ein Endoskop vorgesehen ist.

7. Vorrichtung nach Anspruch 1, wobei als chirurgisches Therapie- und/oder Diagnoseinstrument eine Biopsienadel vorgesehen ist.

8. Vorrichtung nach Anspruch 1, wobei der Adapter (3) am Gehäuse (2.1) des Operationsmikroskopes (2) lösbar angeordnet ist und eine zylinderförmige Führung aufweist, in die das jeweilige chirurgische Therapieund/oder Diagnoseinstrument einführbar ist, wobei die Achse der zylinderförmigen Führung in die Richtung der Sehfeldmitte ausgerichtet ist.

9. Vorrichtung nach Anspruch 8, wobei der Adapter (3) mindestens eine einstellbare mechanische Endlage aufweist, die ein Verschieben des jeweiligen chirurgischen Therapie- und/oder Diagnoseinstrumentes in Adapter-Längsrichtung in der zylinderförmigen Führung nur bis zu einer definierten Position erlaubt.

10. Vorrichtung nach Anspruch 1, wobei das verwendete Operationsmikroskop (2) ein Positionserkennungssystem auf optischer oder akustischer Basis umfaßt.

11. Vorrichtung nach einem der Ansprüche 1 bis 10, wobei das Trägersystem ein von der zentralen Steuereinheit (3) gesteuertes motorisches Trägersystem 1 ist.

12. Stereotaktischer Adapter für eine Vorrichtung nach einem der Ansprüche 1 bis 11 zur Anordnung eines chirurgischen Therapie- und/oder Diagnoseinstrumentes an dem Operationsmikroskop (2), wobei der Adapter (3) eine reproduzierbare Anordung des jeweiligen chirurgischen Therapie- und/oder Diagnoseinstrumentes am beobachtungswirksamen Operationsmikroskop (2) gewährleistet.

## Claims

1. Apparatus for computer-assisted surgery comprising a surgical microscope (2) with a position detection system, a suspension system (1) carrying the surgical microscope (2), which allows for positioning of the surgical microscope (2) in at least three spatial degrees of freedom, and a central control unit,
**characterized by**
a stereotactic adapter (3) for mounting a surgical therapy and/or diagnosis instrument on the surgical microscope (2), with the adapter (3) guaranteeing the reproducible arrangement of the respective therapy and/or diagnosis instrument to the surgical microscope (2) while the surgical microscope (2) allows for constant viewing.

2. Apparatus according to claim 1, whereby the adpater (3) and/or the surgical microscope (2) has a code recognition system which identifies the code of the surgical therapy and/or diagnosis instrument used and transmits this information to the central control unit, with the control unit clearly identifying the instrument and the instrument's geometric data using the information transmitted.

3. Apparatus according to claim 2, whereby an optical coding system is provided.

4. Apparatus according to claim 2, whereby a magnetic coding system is provided.

5. Apparatus according to claim 1, whereby the central control unit comprises an input interface for the manual identification of the surgical therapy and/or diagnosis instrument used.

6. Apparatus according to claim 1, whereby an endoscope is provided as surgical therapy and/or diagnosis instrument.

7. Apparatus according to claim 1, whereby a biopsy needle is provided as surgical therapy and/or diagnosis instrument.

8. Apparatus according to claim 1, whereby the adapter (3) is removebally attached to the housing (2.1) of the surgical microscope (2) and having a cylindrical duct into which the surgical therapy and/or diagnosis instrument can be inserted, with the axis of the cylindrical duct pointing in the direction of the field-of-view center.

9. Apparatus according to claim 8, whereby the adapter (3) has at least one adjustable mechanical end stop which allows the movement of the surgical therapy and/or diagnosis instrument in the longitudinal direction of the adapter in the cylindrical duct only up to a defined position.

10. Apparatus according to claim 1, whereby the surgical microscope (2) used has a position recognition system on an optical or acoustic basis.

11. Apparatus according to one of the claims 1 to 10, whereby the suspension system is a motorized suspension system controlled by the control unit (3).

12. Stereotactic adapter for a device according to one of the claims 1 to 11 for mounting a surgical therapy and/or diagnosis instrument on the surgical microscope (2), with the adapter (3) guaranteeing the reproducible arrangement of the surgical therapy and/or diagnosis instrument on the surgical microscope (2) while the surgical microscope allows for constant viewing.

## Revendications

1. Dispositif pour la chirurgie assistée par ordinateur comprenant
un microscope d'opération (2) muni d'un système de positionnement,
un système (1) supportant le microscope d'opération (2), qui permet de positionner ledit microscope d'opération (2) dans l'espace selon au moins trois degrés de liberté, et
une unité de commande centrale,
**caractérisé par**
un adaptateur stéréotaxique (3) servant à disposer un instrument chirurgical thérapeutique et/ou diagnostique sur le microscope d'opération (2), ledit adaptateur (3) garantissant une disposition reproductible de l'instrument chirurgical thérapeutique et/ou diagnostique sur le microscope d'opération (2) disponible pour l'observation.

2. Dispositif selon la revendication 1, où l'adaptateur (3) et/ou le microscope d'opération (2) sont dotés d'un système de reconnaissance de code qui identifie un codage de l'instrument chirurgical thérapeutique et/ou diagnostique adapté et transmet ces informations à l'unité de commande centrale, cette unité de commande centrale identifiant l'instrument en question et sa géométrie sur la base des informations ainsi transmises.

3. Dispositif selon la revendication 2, où un système de codage optique est prévu.

4. Dispositif selon la revendication 2, où un système de codage magnétique est prévu.

5. Dispositif selon la revendication 1, où l'unité de commande centrale comprend une interface d'entrée pour l'identification manuelle de l'instrument chirurgical thérapeutique et/ou diagnostique utilisé.

6. Dispositif selon la revendication 1, où 1' instrument thérapeutique et/ou diagnostique prévu est un endoscope.

7. Dispositif selon la revendication 1, où l'instrument chirurgical thérapeutique et/ou diagnostique prévu est une aiguille à biopsie.

8. Dispositif selon la revendication 1, où l'adaptateur (3) est disposé de façon détachable sur le boîtier (2.1) du microscope d'opération (2) et présente un guidage de forme cylindrique, dans lequel peut s'engager l'instrument chirurgical thérapeutique et/ou diagnostique, l'axe dudit guidage de forme cylindrique étant orienté en direction du centre du champ de vision.

9. Dispositif selon la revendication 8, où l'adaptateur (3) présente au moins une butée mécanique réglable qui permet de déplacer l'instrument chirurgical thérapeutique et/ou diagnostique dans l'axe longitudinale de l'adaptateur, le long du guidage de forme cylindrique, jusqu'à une position définie de fin de course.

10. Dispositif selon la revendication 1, où le microscope d'opération utilisé (2) comprend un système de positionnement fonctionnant par voie optique ou acoustique.

11. Dispositif selon l'une des revendications 1 à 10, où le système de support 1 est un système motorisé commandé par l'unité de commande centrale (3).

12. Adaptateur stéréotaxique pour un dispositif selon l'une des revendications 1 à 11 servant à disposer un instrument chirurgical thérapeutique et/ou diagnostique sur le microscope d'opération (2), où l'adaptateur (3) garantit une disposition reproductible de l'instrument chirurgical thérapeutique et/ou diagnostique utilisé sur le microscope d'opération (2) disponible pour l'observation.
